# FASCICULE DE BREVET EUROPEEN

(11) **EP 1 254 139 B1**
(45) Date de publication et mention de la délivrance du brevet: **20.08.2003**
(21) Numéro de dépôt: 01907686.8
(22) Date de dépôt: 24.01.2001
(51) Int. Cl.: C07D 487/08, A61K 31/551

(54) **DERIVES DE 4-HETEROARYL-1,4-DIAZABICYCLO 3.2.2]NONANE, LEUR PREPARATION ET LEUR APPLICATION EN THERAPEUTIQUE**
4-HETEROARYL-1,4-DIAZABICYCLO 3.2.2.]NONANE, IHRE HERSTELLUNG UND IHRE THERAPEUTISCHE ANWENDUNG
4-HETEROARYL-1,4-DIAZABICYCLO 3.2.2]NONANE, PREPARATION AND THERAPEUTIC USE THEREOF

(30) Priorité: 28.01.2000 FR 0001098
(43) Date de publication de la demande: 06.11.2002
(73) Titulaire: SANOFI-SYNTHELABO, 75013 Paris (FR)
(72) Inventeur: GALLI, Frédéric, F-92420 Vaucresson (FR); JEGHAM, Samir, F-34980 Montferrier sur Lez (FR); LECLERC, Odile, F-91640 Briis sous Forges (FR); LOCHEAD, Alistair, F-94220 Charenton-Le-Pont (FR); NEDELEC, Alain, F-92700 Colombes (FR)
(74) Mandataire: Ludwig, Jacques
(86) Numéro de dépôt international: FR0100227
(87) Numéro de publication internationale: WO01055150

(56) Documents cités:
- WO-A-00/34279
- WO-A-00/34284
- US-A- 5 478 939

## Description

La présente invention a pour objet des composés répondant à la formule générale (I)
dans laquelle 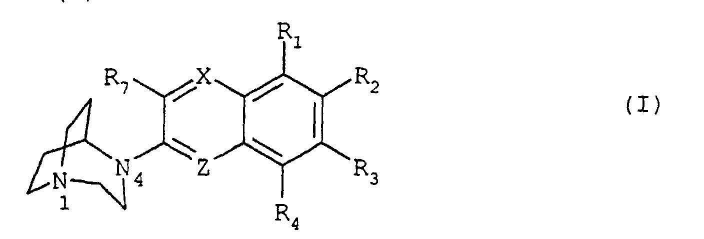
R₁, R₂, R₃ et R₄ représentent chacun, indépendamment les uns des autres, un atome d'hydrogène ou d'halogène ou un groupe nitro, amino, trifluorométhyle, cyano, hydroxy, (C₁-C₆)alkyle ou (C₁-C₆)alcoxy,
X représente
   - soit un atome d'azote, auquel cas Z représente un groupe de formule C-R₅ ou un atome d'azote,
   - soit un groupe de formule C-R₆, auquel cas Z représente un atome d'azote,
R₅ et R₆ représentant chacun, indépendamment l'un de l'autre, un atome d'hydrogène ou d'halogène ou un groupe trifluorométhyle, cyano, hydroxy, (C₁-C₆)alkyle ou (C₁-C₆)alcoxy, et
R₇ représente un atome d'hydrogène ou un groupe (C₁-C₆)alkyle.

Les composés de l'invention peuvent exister à l'état de bases ou de sels d'addition à des acides.

Conformément à l'invention, on peut préparer les composés de formule générale (I) par un procédé illustré par le schéma qui suit.

On fait réagir le 1,4-diazabicyclo[3.2.2]nonane de formule (II) avec un composé hétérocyclique de formule générale (III) dans laquelle R₁, R₂, R₃, R₄, R₇, X et Z sont tels que définis ci-dessus et W représente un atome d'halogène. On peut ainsi effectuer une réaction de couplage de type Buchwald (*J. Org. Chem*. 1997, **62**, 6066-6068) en présence d'un catalyseur au palladium tel que l'acétate de palladium, le tris(dibenzylidèneacétone)dipalladium (0), etc, d'un ligand de complexation tel que la triphénylphosphine, la tributylphosphine ou le 2,2'-bis(diphénylphosphino)-1,1'-binaphtyle, et d'une base, par exemple organique telle que le t-butoxyde de sodium, ou minérale telle que le carbonate de césium.

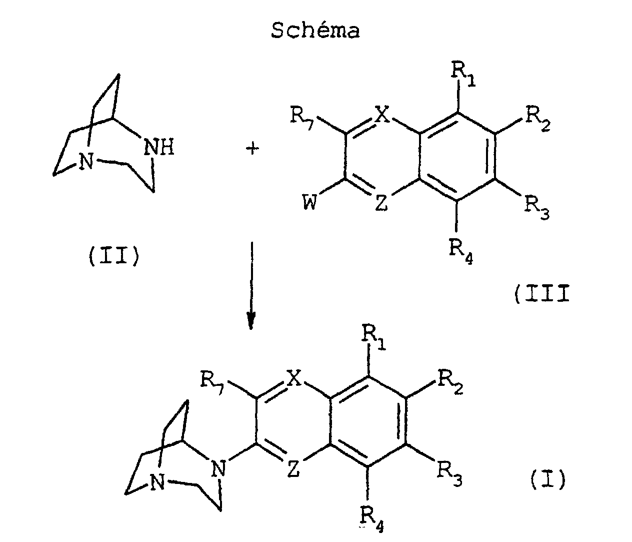

On peut aussi effectuer une réaction de substitution nucléophile en présence d'une base forte telle que le carbonate de césium ou la triéthylamine.

La préparation du 1,4-diazabicyclo[3.2.2]nonane est décrite dans *J. Med. Chem.* 1993, **36**, 2311-2320.

Les composés de formule générale (III) sont disponibles dans le commerce ou sont accessibles par des méthodes décrites dans la littérature.

Les exemples qui vont suivre illustrent la préparation de quelques composés de l'invention. Les microanalyses élémentaires, et les spectres IR et RMN confirment les structures des composés obtenus. Les numéros indiqués entre parenthèses dans les titres des exemples correspondent à ceux de la 1ère colonne du tableau donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°1).

### Bromhydrate de 4-(quinoléin-3-yl)-1,4-diazabicyclo[3.2.2]_ nonane 2:1

Dans un ballon tricol de 500 ml on introduit successivement 2,3 g (18 mmoles) de 1,4-diazabicyclo[3.2.2]nonane, 11,3 g (55 mmoles) de 3-bromoquinoléine, 8,3 g (25 mmoles) de carbonate de césium, 0,164 g (0,73 mmoles) de diacétate de palladium et 0,454 g (0,73 mmoles) de 2,2'-bis(diphényl_ phosphino)-1,1'-binaphtyle, en solution dans 180 ml de tétrahydrofurane, et on chauffe le mélange réactionnel au reflux pendant 22 h.

On le filtre sur terre d'infusoires, on évapore le solvant sous pression réduite et on purifie le résidu par chromatographie sur colonne de gel de silice en éluant par un mélange 95/5/0,5 de dichlorométhane, méthanol et ammoniaque.

On obtient 0,83 g de produit huileux que l'on traite par 1,15 ml d'une solution à 5,7 M d'acide bromhydrique dans l'acide acétique, et on recristallise les cristaux obtenus dans un mélange d'éthanol et de méthanol.
Point de fusion : 309-316°C.

### Exemple 2 (Composé N°2).

### 4-(8-Nitroquinoléin-3-yl)-1,4-diazabicyclo[3.2.2]nonane.

### 2.1 3-Bromo-8-nitroquinoléine.

Dans un ballon tricol de 500 ml on introduit 10 g (57 mmoles) de 8-nitroquinoléine en solution dans 100 ml d'acide acétique, on ajoute 11,3 g (63 mmoles) de *N*-bromosuccinimide et on chauffe le mélange à 100-110°C pendant 6 h.

Après refroidissement à température ambiante on verse le milieu réactionnel dans 300 ml d'eau, on recueille le précipité par filtration, on le rince à l'eau et on le sèche sous vide. On purifie le résidu par chromatographie sur colonne de gel de silice en éluant par un mélange 50/50 puis 70/30 de dichlorométhane et cyclohexane.
On obtient 12,3 g de produit.
Point de fusion : 123-124°C.

### 2.2. 4-(8-Nitroquinoléin-3-yl)-1,4-diazabicyclo_ [3.2.2]nonane.

Dans un ballon tricol de 100 ml on introduit successivement 1,54 g (6,1 mmoles) de 3-bromo-8-nitroquinoléine, 0,7 g (5,5 mmoles) de 1,4-diazabicyclo[3.2.2]nonane, 0,05 g (0,22 mmole) de diacétate de palladium, 2,5 g (7,7 mmoles) de carbonate de césium et 0,137 g (0,22 mmoles) de 2,2'-bis_ (diphénylphosphino)-1,1'-binaphtyle dans 30 ml de tétrahydrofurane et 10 ml de toluène et on chauffe le mélange réactionnel à 80-90°C pendant 24 h.
On sépare les produits minéraux par filtration et on évapore le solvant sous pression réduite. On purifie le résidu par chromatographie sur colonne de gel de silice en éluant par un mélange 95/5/0,5 puis 90/10/1 de dichlorométhane, méthanol et ammoniaque.
On obtient 1,18 g de solide que l'on recristallise dans le méthanol.
Point de fusion : 180-181°C.

### Exemple 3 (Composé N°6).

### 4-(8-Aminoquinoléin-3-yl)-1,4-diazabicyclo[3.2.2]nonane.

Dans un ballon tricol de 25 ml on introduit 0,8 g (2,7 mmoles) de 4-(8-nitroquinoléin-3-yl)-1,4-diazabicyclo_ [3.2.2]nonane en suspension dans un mélange de 8 ml d'eau et 4 ml d'acide acétique, on chauffe à 40°C, on ajoute 0,43 g (7,7 mmoles) de fer, en deux portions et on chauffe le mélange à 50°C pendant 1 h.

on le refroidit à température ambiante, on le filtre sur terre d'infusoires, on évapore le solvant sous pression réduite, et on purifie le résidu par chromatographie sur colonne de gel de silice en éluant par un mélange 90/10/1 de dichlorométhane, méthanol et ammoniaque.
On obtient 0,18 g d'une huile jaune qui cristallise.
Point de fusion : 149-152°C.

### Exemple 4 (Composé N° 5).

### 4-(6-Chloroquinoléin-3-yl)-1,4-diazabicyclo[3.2.2]nonane.

### 4.1 3-Bromo-6-chloroquinoléine.

Dans un ballon tricol de 100 ml on introduit 4,8 g (29 mmoles) de 6-chloroquinoléine en solution dans 50 ml d'acide acétique, on ajoute 5,75 g (32 mmoles) de *N*-bromosuccinimide et on chauffe à 100°C pendant 6h.
On verse le milieu réactionnel sur 100 ml d'eau et extrait au dichlorométhane. On sèche les phases organiques réunies sur sulfate de magnésium, on les concentre sous pression réduite et on purifie le résidu par chromatographie sur colonne de gel de silice en éluant par un mélange 50/50 puis 30/70 de cyclohexane et dichlorométhane.
On obtient 4,86 g de produit.
Point de fusion : 110-111°C.

### 4.2 4-(6-Chloroquinoléin-3-yl)-1,4-diazabicyclo[3.2.2]-nonane

Dans un ballon tricol de 50 ml on introduit successivement 0,53 g (2,18 mmoles) de 3-bromo-6-chloroquinoléine, 0,25 g (2 mmoles) de 1,4-diazabicyclo[3.2.2]nonane, 0,018 g (0,08 mmole) de diacétate de palladium, 0,91 g (2,8 mmoles) de carbonate de césium et 0,05 g (0,08 mmoles) de 2,2'-bis(di-phénylphosphino)-1,1'-binaphtyle dans 15 ml de tétrahydrofurane et on chauffe le mélange réactionnel au reflux pendant 26 h.

On sépare les produits minéraux par filtration, on évapore le solvant sous pression réduite et on purifie le résidu par chromatographie sur colonne de gel de silice en éluant par un mélange 95/5/0,5 puis 90/10/1 de dichlorométhane, méthanol et ammoniaque.
On obtient 0,40 g de solide que l'on recristallise dans l'éther diisopropylique.
Point de fusion : 134-135°C.

### Exemple 5 (Composé N°9).

### Bromhydrate de 4-(quinoléin-2-yl)-1,4-diazabicyclo_ [3.2.2]nonane 1:1.

Dans un ballon tricol de 100 ml on introduit successivement 0,83 g (6,58 mmoles) de 1,4-diazabicyclo[3.2.2]nonane et 1,08 g de 2-chloroquinoléine en solution dans 30 ml de toluène et on chauffe le mélange au reflux pendant 72 h.
On concentre la solution sous pression réduite et on purifie le résidu par chromatographie sur colonne de gel de silice en éluant par un mélange 95/5/0,5 puis 90/10/1 de dichlorométhane, méthanol et ammoniaque.

On obtient 0,24 g de produit huileux que l'on met en solution dans l'alcool isopropylique avant d'ajouter 0,17 ml d'une solution d'acide bromhydrique 5,7 N dans l'acide acétique.
Après refroidissement on collecte les cristaux obtenus par filtration et on les sèche sous vide.
Point de fusion : 253-255°C.

Le tableau qui suit illustre les structures chimiques et les propriétés physiques de quelques composés de l'invention.

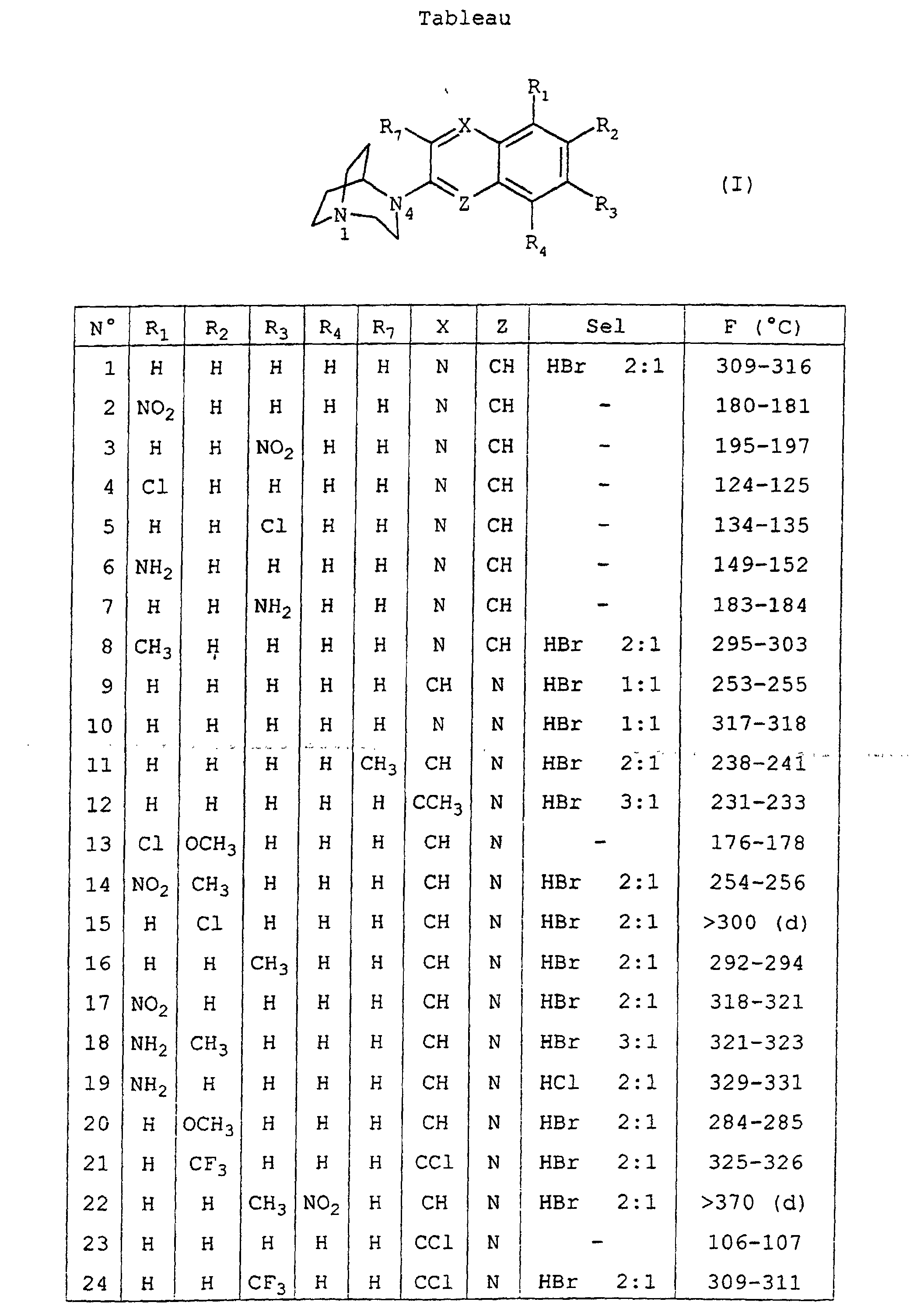

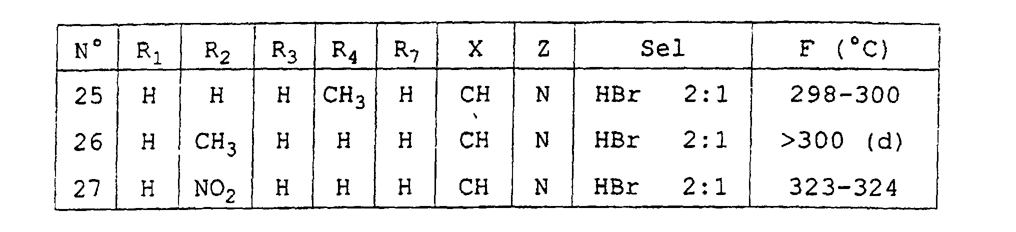

Dans la colonne "Sel", "-" désigne un composé à l'état de base, et "HBr" désigne un bromhydrate ; le rapport molaire acide:base est indiqué en regard.

Dans la colonne "F (°C)", "(d)" indique une température de fusion avec décomposition.

Les composés de l'invention ont fait l'objet d'essais biologiques qui ont mis en évidence leur intérêt comme substances à visées thérapeutiques.

Ainsi ils ont été étudiés quant à leur affinité vis-à-vis des récepteurs nicotiniques contenant la sous unité α₄β₂ selon les méthodes décrites par Anderson et Arneric dans *Eur. J. Pharmacol*. 1994, **253**, 261 et par Hall et coll. dans *Brain Res*. 1993, **600**, 127.

On décapite des rats mâles Sprague Dawley de 150 à 200 g, on prélève rapidement la totalité du cerveau, on l'homogénéise dans 15 volumes d'une solution de sucrose à 0,32 M à 4°C puis on le centrifuge à 1000 g pendant 10 min. On élimine le culot et on centrifuge le surnageant à 20000 g pendant 20 min à 4°C. On récupère le culot et on l'homogénéise à l'aide d'un broyeur Polytron™ dans 15 volumes d'eau bidistillée à 4°C, puis on le centrifuge à 8000 g pendant 20 min. On élimine le culot et on centrifuge le surnageant et la couche de peau ("buffy coat") à 40000 g pendant 20 min, on récupère le culot, on le remet en suspension dans 15 ml d'eau bidistillée et on le centrifuge encore une fois à 40000 g avant de le conserver à -80°C. Le jour de l'expérience on décongèle lentement le tissu et on le met en suspension dans 3 volumes de tampon. On fait incuber 150 µl de cette suspension membranaire à 4°C pendant 120 min en présence de 100 µl de [³H]-cytisine à 1 nM dans un volume final de 500 µl de tampon, en présence ou en absence de composé à tester. On arrête la réaction par filtration sur des filtres Whatman GF/B™ préalablement traités avec de la polyéthylènimine. On rince les filtres avec deux fois 5 ml de tampon à 4°C, et on mesure la radioactivité retenue sur le filtre par scintigraphie liquide. On détermine la liaison non spécifique en présence de (-)-nicotine à 10 µM; la liaison non spécifique représente 75 à 85% de la liaison totale récupérée sur le filtre. Pour chaque concentration de composé étudié on détermine le pourcentage d'inhibition de la liaison spécifique de [³H]-cytisine, puis on calcule la CI₅₀, concentration de composé qui inhibe 50% de la liaison spécifique.

Les CI₅₀ des composés de l'invention les plus affins se situent entre 0,003 et 0,012 µM.

Les composés de l'invention ont aussi été étudiés quant à leur affinité vis à vis des récepteurs nicotiniques contenant la sous unité α₇, selon les méthodes décrites par Mark et Collins dans *J. Pharmacol. Exp. Ther*. 1982, **22**, 564 et par Marks et coll. dans *Mol. Pharmacol*. 1986, **30**, 427. On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4 °C, puis on le centrifuge à 1000 g pendant 10 min. On élimine le culot et on centrifuge le surnageant à 8000 g pendant 20 min à 4 °C. On récupère le culot et on l'homogénéise à l'aide d'un broyeur Polytron™ dans 15 volumes d'eau bidistillée à 4°C, puis on le centrifuge à 8000 g pendant 20 min. On élimine le culot et on centrifuge le surnageant et la couche de peau ("buffy coat") à 40000 g pendant 20 min. On récupère le culot, on le remet en suspension avec 15 volumes d'eau bidistillée à 4°C et on le centrifuge encore une fois à 40000 g pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [³H]-α-bungarotoxine à 1 nM dans un volume final de 250 µl de tampon HEPES 20 mM, polyéthylènimine 0,05%. On arrête la réaction par filtration sur des filtres Whatman GF/C™ préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de α-bungarotoxine à 1 µM finale; la liaison non spécifique représente environ 60 % de la liaison totale récupérée sur le filtre. Pour chaque concentration de composé étudié on détermine le pourcentage d'inhibition de la liaison spécifique de [³H]-α-bungarotoxine, puis on calcule la CI₅₀, concentration de composé qui inhibe 50% de la liaison spécifique.

Les CI₅₀ des composés de l'invention les plus affins se situent entre 0,022 et 5,5 µM.

Les résultats qui précèdent montrent que certains composés de l'invention sont des ligands sélectifs pour les sous unités α₄β₂ ou α₇ du récepteur nicotinique et que d'autres sont mixtes α₄β₂ et α₇.

Enfin les composés de l'invention ont fait l'objet d'essais qui ont mis en évidence leurs propriétés analgésiques. Ainsi ils ont été étudiés dans le modèle de la plaque chauffante, selon la méthode décrite par Eddy et Leimbach dans J. *Pharmacol. Exp. Ther*. 1953, **107**, 385 dans le but de rechercher et quantifier un éventuel effet analgésique.

Des souris de 20 à 30 g sont soumises à un stimulus thermique par contact des pattes avec une plaque maintenue à la température constante de 57,5°C par un bain-marie thermostaté. On mesure le temps de réaction à la douleur qui se manifeste par un léchage des pattes ou un saut. Ainsi, après le délai de prétraitement effectué par voie sous-cutanée ou orale (chaque lot étant constitué de huit animaux pour un même prétraitement), les souris sont déposées individuellement sur la plaque et le temps de réaction à la douleur est mesuré. L'animal est retiré de la plaque immédiatement après la manifestation de la douleur. Le temps maximum d'exposition au stimulus est de 30 secondes. On exprime pour chaque lot le temps moyen de réaction accompagné de l'erreur standard à la moyenne (e.s.m.). Une analyse de variance non paramétrique (Kruskal-Wallis), est effectuée sur l'ensemble du lot. Un test de Wilcoxon permet la comparaison de chaque lot traité au lot témoin. Les différences sont considérées comme statistiquement significatives au seuil de 5%. Ce temps de réaction est significativement augmenté par les analgésiques principalement à effets centraux.
Les composés de l'invention montrent une activité dans ce test aux doses comprises entre 0,3 et 100 mg/kg par voie sous-cutanée ou orale.

Les résultats des divers essais suggèrent l'utilisation des composés dans le traitement ou la prévention des désordres liés à un dysfonctionnement des récepteurs nicotiniques, notamment au niveau du système nerveux central ou du système gastro-intestinal.

Au niveau système nerveux central, ces désordres comprennent les altérations cognitives, plus spécifiquement mnésiques, mais également attentionnelles, liées à la maladie d'Alzheimer, au vieillissement pathologique (Age Associated Memory Impairment,AAMI), au syndrome Parkinsonien, à la trisomie 21 (Down's syndrome), au syndrome alcoolique de Korsakoff, aux démences vasculaires (multi-infarct dementia, MDI), aux déficits de l'attention/hyperactivité (ADHA).

Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.

Les composés de l'invention peuvent également constituer un traitement symptomatique et/ou étiologique des maladies neurodégénératives aiguës ou chroniques. Ils peuvent être utilisés dans les cas de pathologies psychiatriques : schizophrénie, dépression, anxiété, attaques de panique, comportements compulsifs et obsessionnels.

Ils peuvent prévenir les symptomes dus au sevrage au tabac, à l'alcool, aux différentes substances induisant une dépendance, telles que cocaïne, LSD, cannabis, benzodiazepines.

Enfin ils peuvent être utiles pour le traitement de la douleur.

Au niveau du système gastro-intestinal les composés de l'invention pourraient être utiles dans le traitement de la maladie de Crohn, de la colite ulcéreuse, du syndrome du colon irritable et de l'obésité.

Ainsi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables. Les dits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.

Lesdites formes unitaires sont dosées pour permettre une administration journalière de 0,01 à 20 mg de principe actif par kg de poids corporel.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.

Les techniques de réalisation peuvent être la compression 14 directe, la granulation sèche, la granulation humide ou la fusion à chaud.

Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.

Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de mêmes qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé répondant à la formule générale (I) 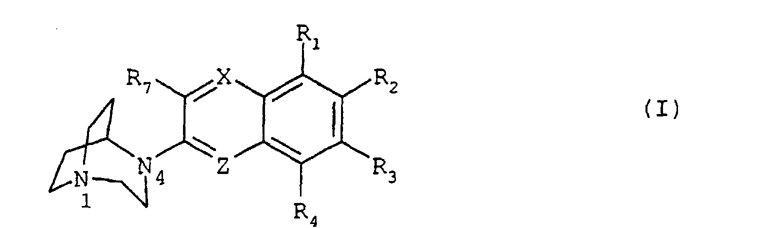 dans laquelle
R₁, R₂, R₃ et R₄ représentent chacun, indépendamment les uns des autres, un atome d'hydrogène ou d'halogène ou un groupe nitro, amino, trifluorométhyle, cyano, hydroxy, (C₁-C₆) alkyle ou (C₁-C₆) alcoxy,
X représente
- soit un atome d'azote, auquel cas Z représente un groupe de formule C-R₅ ou un atome d'azote,
- soit un groupe de formule C-R₆, auquel cas Z représente un atome d'azote,
R₅ et R₆ représentant chacun, indépendamment l'un de l'autre, un atome d'hydrogène ou d'halogène, un groupe trifluorométhyle, cyano, hydroxy, (C₁-C₆) alkyle ou (C₁-C₆) alcoxy, et
R₇ représente un atome d'hydrogène ou un groupe (C₁-C₆)alkyle,
à l'état de base ou de sel d'addition à un acide.

2. Procédé de préparation de composés selon la revendication 1, **caractérisé en ce qu'**on fait réagir le 1,4-diazabicyclo[3.2.2]nonane avec un composé hétérocyclique de formule générale (III) 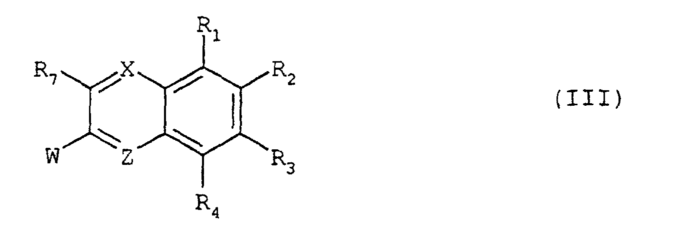 dans laquelle R₁, R₂, R₃, R₄, R₇, X et Z sont tels que définis dans la revendication 1 et W représente un atome d'halogène.

3. Médicament **caractérisé en ce qu'**il consiste en un composé selon la revendication 1.

4. Composition pharmaceutique **caractérisée en ce qu'**elle contient un composé selon la revendication 1, associé à un excipient.

## Patentansprüche

1. Verbindung der allgemeinen Formel (I) 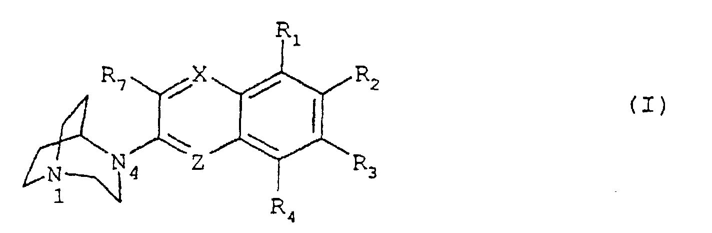 in der
R₁, R₂, R₃ und R₄ jeweils unabhängig voneinander ein Wasserstoffatom oder ein-Halogenatom oder eine Nitrogruppe, Aminogruppe, Trifluormethylgruppe, Cyanogruppe, Hydroxygruppe, (C₁-C₆)-Alkylgruppe oder (C₁-C₆)-Alkoxygruppe bedeuten,
X
- entweder ein Stickstoffatom, wobei in diesem Fall Z eine Gruppe der Formel C-R₅ oder ein Stickstoffatom darstellt;
- oder eine Gruppe der Formel C-R₆, wobei in diesem Fall Z ein Stickstoffatom darstellt, bedeutet,
R₅ und R₆ jeweils unabhängig voneinander ein Wasserstoffatom oder Halogenatom, eine Trifluormethylgruppe, Cyanogruppe, Hydroxygruppe, (C₁-C₆)-Alkylgruppe oder (C₁-C₆)-Alkoxygruppe bedeuten und
R₇ ein Wasserstoffatom oder eine (C₁-C₆)-Alkylgruppe bedeutet,
in Form der Base oder eines Additionssalzes mit einer Säure.

2. Verfahren zur Herstellung der Verbindungen nach Anspruch 1, **dadurch gekennzeichnet, daß** man 1,4-Diazabicyclo[3.2.2]nonan mit einer heterocyclischen Verbindung der allgemeinen Formel (III) 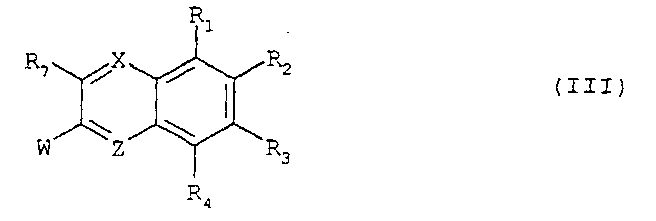 in der R₁, R₂, R₃, R₄, R₇, X und Z die in Anspruch 1 angegebenen Bedeutungen besitzen und W ein Halogenatom darstellt, umsetzt.

3. Arzneimittel, **dadurch gekennzeichnet, daß** es aus einer Verbindung nach Anspruch 1 besteht.

4. Pharmazeutische Zubereitung, **dadurch gekennzeichnet, daß** sie eine Verbindung nach Anspruch 1 in Kombination mit einem Trägermaterial enthält.

## Claims

1. Compound corresponding to the general formula (I) 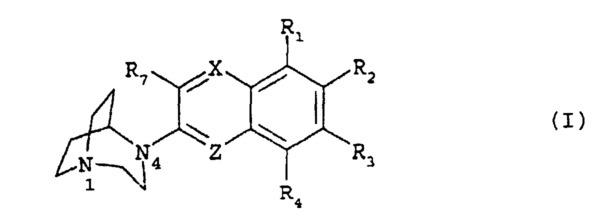 in which
R₁, R₂, R₃ and R₄ each represent, independently of each other, a hydrogen or halogen atom or a nitro, amino, trifluoromethyl, cyano, hydroxyl, (C₁-C₆)alkyl or (C₁-C₆)alkoxy group,
X represents
- either a nitrogen atom, in which case Z represents a group of the formula C-R₅ or a nitrogen atom,
- or a group of the formula C-R₆, in which case Z represents a nitrogen atom,
R₅ and R₆ each represent, independently of each other, a hydrogen or halogen atom or a trifluoromethyl, cyano, hydroxyl, (C₁-C₆)alkyl or (C₁-C₆)alkoxy group, and
R₇ represents a hydrogen atom or a (C₁-C₆)alkyl group,
as a base or an acid addition salt.

2. Process for preparing compounds according to Claim 1, **characterized in that** 1,4-diazabicyclo[3.2.2]nonane is reacted with a heterocyclic compound of the general formula (III) 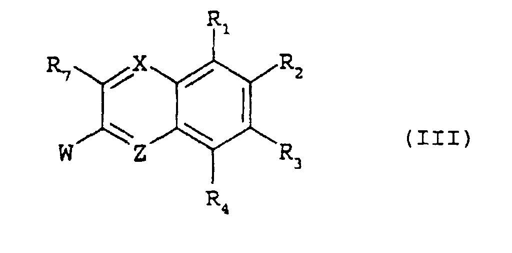 in which R₁, R₂, R₃, R₄, R₇, X and Z are as defined in Claim 1 and W represents a halogen atom.

3. Medicament, **characterized in that** it consists of a compound according to Claim 1.

4. Pharmaceutical composition, **characterized in that** it comprises a compound according to Claim 1 combined with an excipient.
